# EUROPÄISCHE PATENTSCHRIFT

(11) **EP 3 069 700 B1**
(45) Veröffentlichungstag und Bekanntmachung des Hinweises auf die Patenterteilung: **18.03.2020**
(21) Anmeldenummer: 16164607.0
(22) Anmeldetag: 28.06.2007
(51) Int. Cl.: A61F 9/01, A61F 9/008, A61F 9/009

(54) **BESTRAHLUNGSSYSTEM FÜR OPHTHALMOLOGISCHE ANWENDUNGEN**
RADIATION SYSTEM FOR OPHTHALMOLOGICAL APPLICATIONS
SYSTEME DE RAYONNEMENT POUR DES APPLICATIONS OPHTALMOLOGIQUES

(30) Priorität: 30.06.2006 DE 102006030219
(43) Veröffentlichungstag der Anmeldung: 21.09.2016
(62) Teilanmeldung aus: 07764922.6
(73) Patentinhaber: Avedro, Inc., Waltham, MA 02451 (US)
(72) Erfinder: Mrochen, Michael, 8193 Eglisau (CH); Büeler, Michael, 8048 Zürich (CH); Schelling, Urs, 8005 Zürich (CH)
(74) Vertreter: Mewburn Ellis LLP

(56) Entgegenhaltungen:
- EP-A1- 1 285 679
- WO-A-93/16631
- WO-A-94/03134
- WO-A-03/061696
- SOBOL E N ET AL: "CORRECTION OF EYE REFRACTION BY NONABLATIVE LASER ACTION ON THERMOMECHANICAL PROPERTIES OF CORNEA AND SCLERA", QUANTUM ELECTRONICS, TURPION LTD., LONDON, GB, Bd. 32, Nr. 10, Oktober 2002 (2002-10), Seiten 909-912, XP001170947, ISSN: 1063-7818

## Beschreibung

Die Erfindung betrifft ein Bestrahlungssystem für ophthalmologische Anwendungen zur Erzielung von Veränderungen der biomechanischen Eigenschaften von Komponenten des Auges, insbesondere der Hornhaut. Hierfür setzt die vorliegende Erfindung eine hier mit "Primärstrahlung" bezeichnete elektromagnetische Strahlung ein, bevorzugt im spektralen Bereich von 300 nm bis 800 nm. Diese Strahlung wird bevorzugt mit LEDs oder Laserdioden erzeugt. Die hier mit "Primärstrahlung" bezeichnete elektromagnetische Strahlung soll keinen sogenannten fotoablativen Effekt bewirken, also keinen Effekt, bei dem Gewebe aus dem Auge entfernt wird, wie dies bei einer Neuformung der Kornea zum Beispiel gemäß der LASIK, erfolgt. Vielmehr dient die erfindungsgemäße Primärstrahlung dazu, das Gewebe, insbesondere die Hornhaut, in ihren biomechanischen Eigenschaften zu verändern, ohne Gewebe zu entfernen. Eine Änderung der biomechanischen Eigenschaften zum Beispiel der Hornhaut liegt dann vor, wenn das Gewebe in seiner Elastizität verändert wird ("verhärtet"). Hierzu kennt der Stand der Technik sogenannte Fotosensibiliatoren, also Wirkstoffe, die in das Gewebe injiziert werden, und die dort den genannten Effekt der Änderung biomechanischer Eigenschaften des Gewebes fördern. Die erfindungsgemäße Primärstrahlung bewirkt also im Ergebnis eine biomechanische Stabilisierung der Hornhaut.

Die Druckschrift WO 93/16631 beschreibt ein Bestrahlungssystem für Ablation oder Fotokoagulation von Hornhautgewebe. Die fotomechanische Stabilisierung von Hornhautgewebe, in die ein Fotosensibilisator eingebracht ist, ohne fotoablativen Effekt, findet jedoch mit Strahlungsintensitäten statt, die um mehrere Zehnerpotenzen verschieden sind von Strahlungsintensitäten, wie sie für eine Fotoablation oder Fotokoagulation eingesetzt werden.

Der Erfindung liegt die Aufgabe zugrunde, ein für die fotomechanische Stabilisierung von Hornhautgewebe, ohne fotoablativen Effekt, optimales Bestrahlungssystem bereitzustellen.

Hierzu dient das Bestrahlungssystem gemäß Anspruch 1.

Weitere Ausgestaltungen der Erfindung sind in den weiteren Ansprüchen und der nachfolgenden Beschreibung von Ausführungsbeispielen anhand der Figuren beschrieben.

Es zeigt:
- Figur 1: schematisch ein Bestrahlungssystem für medizinische Anwendungen;
- Figur 2: eine Einzelheit des Bestrahlungssystems;

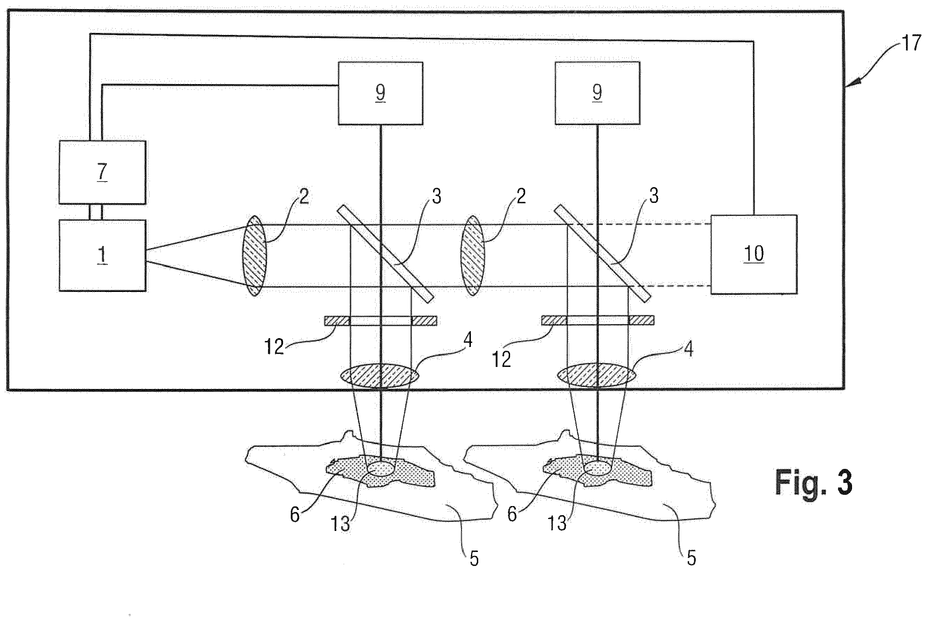

- Figur 3: eine weitere Ausführungsform eines Bestrahlungssystems für medizinische Zwecke mit zwei Subsystemen;
- Figur 4: schematisch Ausführungsbeispiele für Bestrahlungsfelder;
- Figur 5: eine weitere Einzelheit eines Bestrahlungssystems für medizinische Zwecke mit einer Kalibriereinrichtung;
- Figur 6: eine weitere Einzelheit eines Bestrahlungssystems für medizinische Zwecke;
- Figur 7: schematisch einen Adapter für ein Bestrahlungssystem der vorstehend genannten Art zur Positionierung in Bezug auf ein Auge;
- Figur 8: ein weiteres Ausführungsbeispiel für eine Adapter ähnlich Figur 7;

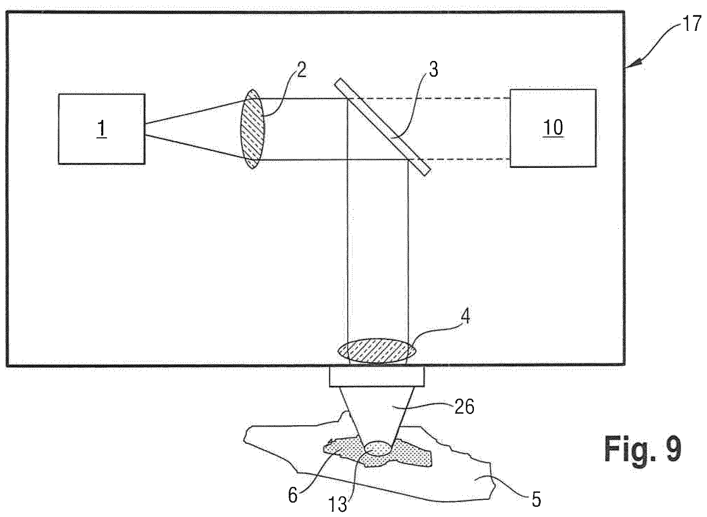

- Figur 9: das Zusammenwirken eines medizinischen Strahlungssystems mit einem Adapter;
- Figur 10: eine Zentrierung von Bestrahlungsmitteln;
- Figur 11: ein Behandlungssystem mit Einstelleinrichtungen für optische Komponenten;
- Figur 12: eine Variante der Ausführungsform von Figur 11;
- Figur 13: eine Variante von Figur 12 und
- Figur 14: eine Einrichtung zum Austausch von optischen Elementen.

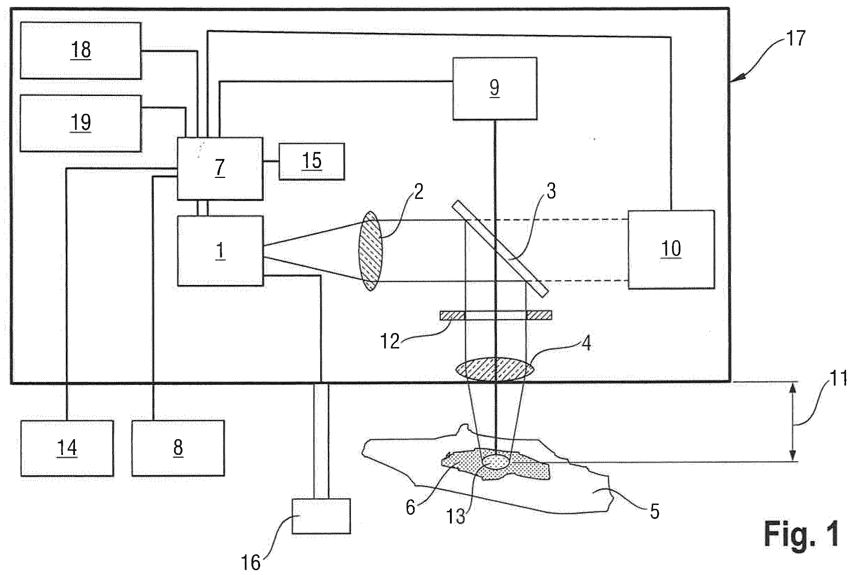

Das Bestrahlungssystem für medizinische Zwecke gemäß Figur 1 weist eine Strahlungsquelle 1 für elektromagnetische Strahlung auf, zum Beispiel eine LED, einen Laser, oder eine thermische Lichtquelle. Die Strahlung wird über eine Linse 2 und einen halbdurchlässigen Spiegel 3 sowie einer weiteren Linse 4 auf zu behandelndes Gewebe 5 fokussiert.

Die hier beschriebenen Ausführungsbeispiele der Erfindung eignen sich insbesondere für den ophthalmologischen Einsatz des Bestrahlungssystems.

In das zu behandelnde Gewebe 5 ist ein Wirkstoff eingebracht, der die fotochemischen und/oder fotophysikalischen Wirkungen der in das Gewebe eingebrachten Strahlung ermöglicht oder fördert.

Der Bestrahlungsbereich ist mit dem Bezugszeichen 13 bezeichnet.

Eine Bestrahlungseinheit 17 ist auf einem Stativ 16 so abgestützt, dass ein vorgegebener Abstand 11 zwischen dem im Strahlungsweg zuletzt angeordneten optischen Element 4 der Bestrahlungseinheit 17 und dem zu bestrahlenden Gewebe 5 einstellbar ist.

Mit dem Strahlteiler 3 können wahlweise unterschiedliche Funktionen erreicht werden:
Zum Einen kann mit dem Strahlteiler 3 ein Teil der von der Strahlungsquelle 1 emittierten Strahlung ausgekoppelt und einer Messeinrichtung 10 zugeführt werden, die zum Beispiel die Energie und/oder die Intensitätsverteilung und/oder die Zeitverteilung der Strahlung misst.

Andererseits kann mit dem Strahlteiler 3 auch ein Teil der vom Gewebe 5 zurückreflektierten Strahlung einer Beobachtungseinrichtung 9 zugeführt werden.

Gemäß einer dritten Variante kann mit dem Strahlteiler 3 die von der Strahlungsquelle 1 emittierte Strahlung mit einer weiteren Strahlung kombiniert werden, wobei die weitere Strahlung dann von einer weiteren Strahlungsquelle emittiert wird, die im mit dem Bezugszeichen 9 versehenen Bauteil angeordnet ist. Die weitere Strahlung hat dann vorzugsweise eine andere Wellenlänge als die von der Strahlungsquelle 1 emittierte Strahlung.

Eine Steuerung 7 dient zur Steuerung u.A. der genannten Komponenten 1, 10 und 9.

Die Steuerung 7 erhält Daten von einem Sensor 15, der wichtige Parameter des Bestrahlungssystems erfasst, wie zum Beispiel die Stromaufnahme der Strahlungsquelle, Temperaturen des Systems und/oder der Umgebung, die Luftfeuchtigkeit der Umgebung und weitere Größen.

Ein Netzteil 14 dient der Energieversorgung der Bestrahlungseinheit 17.

Ein Anzeigelement 18 dient zur Anzeige interessierender Parameter, wie der Lichtemission, interessierender Behandlungsparameter bezüglich des Patienten, oder auch möglicher auftretender Fehler.

Eine Schnittstelle 8 ist mit der Steuerung 7 verbunden und dient dem Anschluss derselben an einen externen Rechner (nicht gezeigt), zum Übertragen von behandlungsrelevanten Daten, wie zum Beispiel den Bestrahlungsdauern, der Bestrahlungsdosis, der Lichtverteilung, interessierenden Messdaten, oder auch zur Übertragung von Daten aus einer Datenbank.

Eine Schnittstelle 19 kann vorgesehen sein zum Übermitteln von Daten an die Steuerung 7 und insbesondere zum Anschluss eines PC mit Eingabeeinrichtungen für den Benutzer bezüglich der Einzelheiten der Behandlung.

In den Figuren sind einander entsprechende oder funktionsähnliche Bauteile mit den gleichen Bezugszeichen versehen.

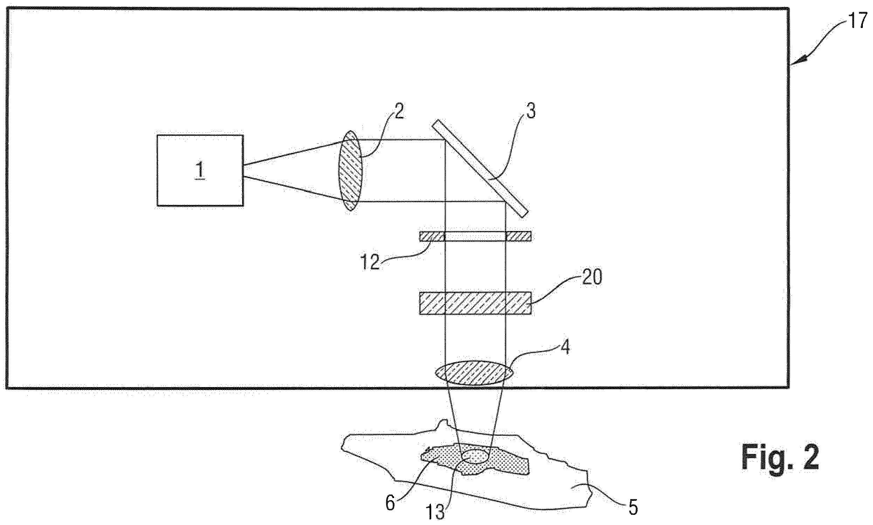

Figur 2 zeigt eine Einzelheit des in Figur 1 dargestellten Bestrahlungssystems, wobei in der Behandlungseinheit 17 im Strahlengang vor der Linse 4 ein optisches Element 20 angeordnet ist, das eine diffraktive oder holografische Wirkung hat, um im Bestrahlungsbereich 13 eine vorgegebene und auswählbare Lichtverteilung zu erzeugen.

In Abwandlung des vorstehend beschriebenen Ausführungsbeispieles kann das optische Element 20 auch ein zeitlich veränderbarer Lichtmodulator sein, zum Beispiel ein Flüssigkristallmodulator, um im Bestrahlungsbereich 13 eine auswählbare Lichtverteilung zu erzeugen.

In Abwandlung der vorstehend beschriebenen Ausführungsbeispiele kann das optische Element 20 auch durch Bewegung im Raum eine einstellbare und veränderliche Lichtverteilung im Gewebe 5 erzeugen.

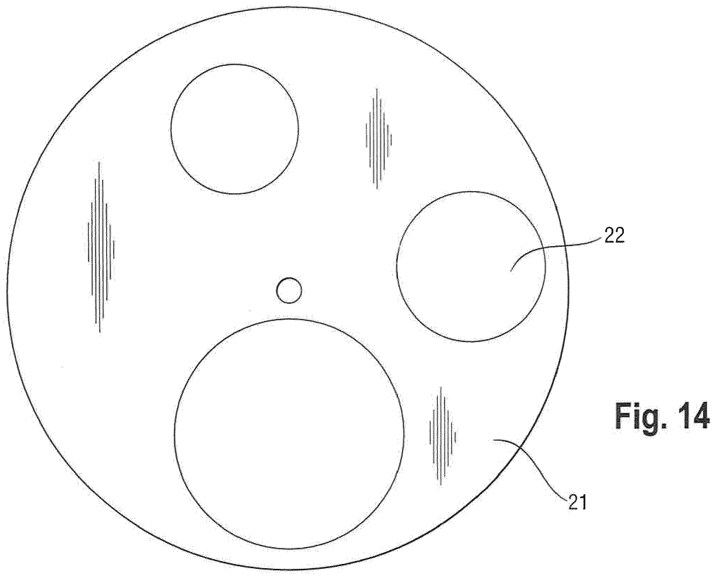

Gemäß einer weiteren Variante kann vorgesehen sein, an der Stelle des optischen Elementes 20 ein in Figur 14 gezeigtes Wechselrad (Revolver) 21 anzuordnen, mit dem unterschiedliche optische Elemente, wie zum Beispiel Absorber 22 in den Strahlungsweg bewegbar sind, um eine vorgegebene Lichtverteilung im Strahlungsbereich 13 zu erzeugen. Dabei ist in den zu bestrahlenden Bereich des Gewebes 5 ein chemische Wirkstoff 6 (Figur 2) eingebracht, um den physikalischen oder chemischen Effekt der Strahlung zu ermöglichen oder zumindest zu fördern. Solche chemischen Wirkstoffe sind als solche bekannt.

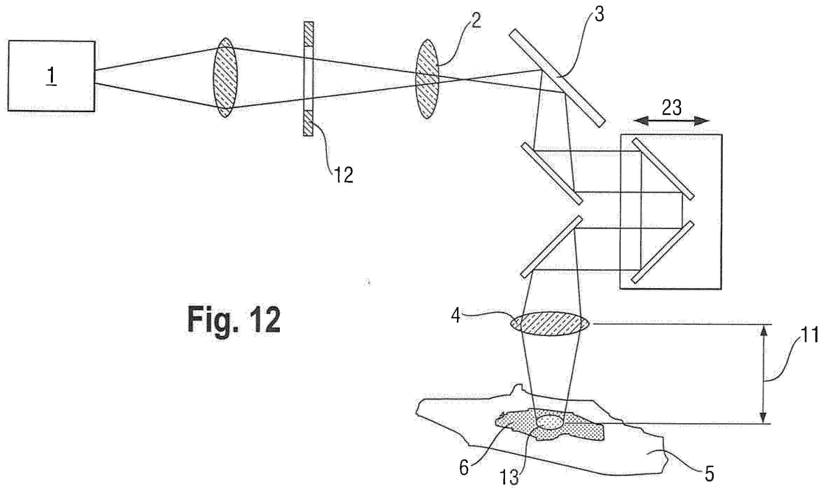

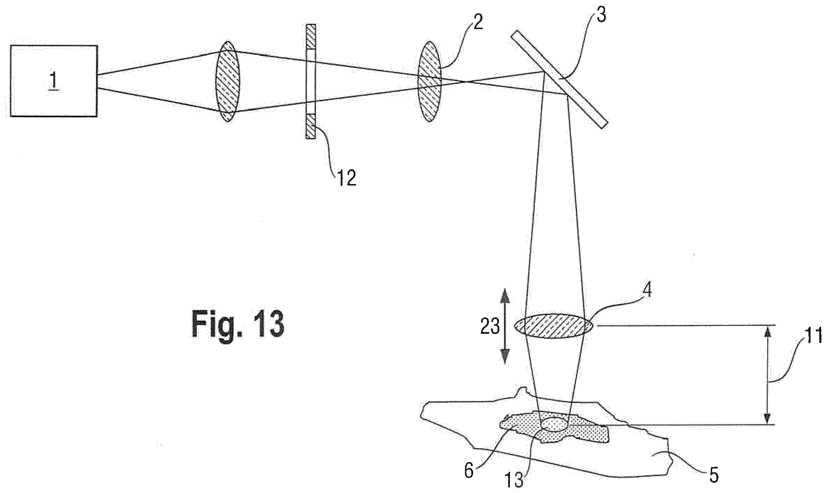

Die Figuren 12 und 13 zeigen Abwandlungen der vorstehend beschriebenen Ausführungsbeispiele, wobei optische Elemente, wie durch den Pfeil 23 angedeutet, im Raum verfahrbar sind, um die Lichtverteilung im Bestrahlungsfeld 13 einzustellen.

Diese Ausführungsbeispiele ermöglichen auch, dass der Abstand 11 zwischen der Bestrahlungseinheit 17 und dem zu bestrahlenden Gewebe 5 einstellbar ist.

Die in Figur 1 gezeigte Blende 12 ist vorzugsweise in ihren Blendenöffnung und/oder hinsichtlich ihres Abstandes in Bezug auf die anderen optischen Elemente steuerbar (einstellbar).

Gemäß einer Variante kann die Blende 12 als eine rotierende Maske ausgestaltet sein derart, dass durch die rotierende Blende (Maske) jeweils bei der Rotation unterschiedliche Bereiche des Strahls ausgeblendet werden, sodass es zu einer zeitlich und räumlich variierenden Strahlungsdosis auf dem Gewebe 5 kommt. So erzeugt zum Beispiel eine spiralförmige Maske eine parabolische Lichtverteilung im Strahlungsbereich 13.

Nachfolgend wird das Bestrahlungssystem hinsichtlich des im Block 9 wahlweise vorgesehenen Diagnosemittels näher beschrieben:
Das Messmittel 9 kann zum Beispiel ein Gerät für die optische Kohärenztomographie sein. Wahlweise kann das Messmittel 9 auch ein Gerät zur Messung der optische Augenlänge oder ein Messmittel zur Ermittlung der Hornhauttopographie in Echtzeit sein.

Eine andere Ausgestaltung sieht vor, dass das Messmittel 9 ein Wellenfrontdiagnosesystem ist zur Messung der Wellenfront in Echtzeit, die vom Gewebe 5 zurückgestrahlt wird.

Wahlweise kann das Messmittel 9 auch eine Scheimpflugkamera sein.

Eine andere Ausgestaltung sieht vor, dass das Messmittel 9 ein Videosystem zur Bildgabe ist. Auch kann das Messmittel 9 ein Kamerasystems zur elektronischen Bildgabe sein.

Eine andere Ausgestaltung sieht vor, dass das Messmittel 9 ein Mikroskop ist zur visuellen Beobachtung der Behandlung.

Andererseits kann das Messmittel 9 ein Spektrometer zur Fluoreszenzanalyse sein.

Wiederum in einer anderen Ausgestaltung kann es sich bei dem Messmittel 9 um ein System zur Erfassung der Augenbewegungen (sogenannter eye tracker) sein.

Eine andere Ausgestaltung sieht vor, dass das Messmittel 9 ein Gerät ist zur Messung der Hornhaut und/oder der Epitheldicke.

Auch kann es sich bei dem Messmittel 9 um ein Gerät zur Abstandsmessung vom Auge (bezogen auf die optischen Komponenten) handeln.

Nachfolgend werden unterschiedliche Funktionen des Stahlteilers 3 gemäß unterschiedlichen Ausführungsbeispielen der Erfindung erläutert:
Wie oben bereits angedeutet, dient der Strahlteiler 3 bei einer ersten Variante der Erfindung dazu, mit der von der Strahlungsquelle 1 kommenden Strahlung (sogenannte Primärstrahlung) eine Strahlung einer anderen Wellenlänge zu kombinieren, wobei die Strahlungsquelle für die zweite Strahlung (Sekundärstrahlung) im mit 9 bezeichneten Block sitzt. Bei der Sekundärstrahlung kann es sich zum Beispiel um Strahlung mit einer Wellenlänge handeln, die zur UV-Fotoablation von Hornhaut geeignet ist.

Gemäß einer anderen Variante der Erfindung kann die Sekundärstrahlung so ausgewählt sein, dass sie im bestrahlten Gewebe eine Fluoreszenzwirkung erzielt, wobei dann Einrichtungen vorgesehen sind, um die Fluoreszenzstrahlung auszuwerten.

Eine weitere Variante des Einsatzes des Strahlteilers 3 sieht vor, dass die Sekundärstrahlung eine Wellenlänge hat, die geeignet ist, um das zu bestrahlende Gewebe 5 thermisch anzuregen und so die gewünschten Effekte zu fördern.

Eine weitere Variante der Funktion des Strahlteilers 3 ist eine Sekundärstrahlung, die so ausgewählt ist, dass sie im sichtbaren optischen Bereich (für den Patienten sichtbar) liegt, und als sogenannter Fixationsstrahl oder Zielstrahl dient.

Gemäß einer anderen Variante dient der Strahlteiler 3 dazu, die Primärstrahlung in zwei Strahlengänge aufzuteilen, sodass ein (kleinerer) Strahlteil zu Messzwecken in eine Messeinrichtung 10 eingegeben werden kann. Dabei wird das Signal der Messeinrichtung 10 in die Steuerung 7 zur Verarbeitung gegeben.

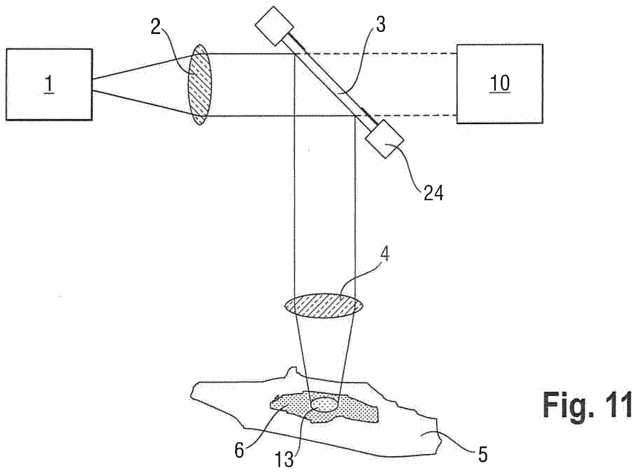

Gemäß einer anderen Variante ist der Strahlteiler 3 durch elektrisch ansteuerbare Mittel 24, siehe Figur 11, so bewegbar, dass der Bestrahlungsbereich 13 über das Gewebe 5 geführt ("gescanned") werden kann.

Nachfolgend werden Einzelheiten der Steuerung 7 erläutert:
Die Steuerung 7 kann so ausgelegt sein, dass sie die genannte Primärstrahlung zeitlich pulsierend abgibt oder kontinuierlich.

Auch kann die Steuerung 7 so programmiert sein, dass die Leistung der primären Strahlung zeitlich variierend einstellbar ist. Dabei sieht eine besondere Ausgestaltung vor, dass die Leistung der von der Quelle 1 abgegebenen Primärstrahlung vor einem eigentlichen Behandlungsbeginn über eine vorgegebene Zeitspanne unterhalb einem vorgegebenen Schwellenwert gehalten wird, um innerhalb dieser vorgegebenen Zeitspanne Justierungen oder Messungen mit der Strahlung durchzuführen. Nach der Zeitspanne kann dann die Strahlung über den genannten Schwellenwert angehoben werden, um eine erwünschte chemische und/oder physikalische Wirkung zu erzielen.

Die Steuerung 7 kann über einen Fusstaster zur Aussendung der Strahlung steuerbar sein. Es ist auch möglich, die Steuerung 7 über eine Fernbedienung zur Aussendung der Strahlung zu bedienen.

Sind mehrere Strahlungsquellen, zum Beispiel mehrere LEDs zur Erzeugung der Primärstrahlung vorgesehen, kann die Steuerung 7 einzelne der Strahlungsquellen jeweils ansteuern, um einen gewünschten räumlichen und/oder zeitlichen Intensitätsverlauf der Strahlung zu steuern.

Der insbesondere eine Messeinrichtung anzeigende Block 10 gemäß Figur 1 ist insbesondere ein Fotodetektor, mit dem die Strahlungsdosis pro Zeiteinheit und über den zeitlichen Verlauf der Behandlung gemessen wird. Dabei kann vorgesehen sein, dass von der Messeinrichtung 10 ein Signal an die Steuerung 7 gegeben wird, um den zeitlichen Verlauf der Strahlung in der Behandlungsebene gemäß einem vorgegebenen Programm zu steuern. Treten Abweichungen hinsichtlich eines gemessenen Parameters gegenüber dem Soll-Verlauf des Programms auf, kann in der Art eines geschlossenen Regelkreises die Steuerung 7 die Strahlung so ändern, dass der genannte Parameter wieder im Soll liegt.

Bezeichnet der Block 9 in Figur 1 einen sogenannten Eye-Tracker, dann kann ein entsprechendes Signal über die Bewegung des Auges an die Steuerung 7 gegeben werden (in den Figuren zeigen die Verbindungslinien zwischen den Funktionsblöcken den wechselseitigen Datenaustausch an) und die Steuerung 7 kann dann einen Motor 24 (Figur 11) betätigen, um den beweglichen Strahlteiler 3 entsprechend der Bewegung des Auges nachzuführen.

Gemäß einer weiteren Ausgestaltung ist die Steuerung 7 so ausgelegt, dass sie das bewegbare Stativ 16 ansteuert und so die Position der Bestrahlungseinheit 17 in Bezug auf das Gewebe 5 einstellt.

Die Steuerung 7 ist so programmiert, dass sie über eine Schnittstelle von z.B. einem Rechner erhaltene Daten, insbesondere bezüglich der Hornhautdicke, der Epitheldicke, der Riboflavinkonzentration (letzteres ist ein Beispiel für eine Wirksubstanz 6 im Gewebe 5) verrechnet, um für die Behandlung optimale Werte hinsichtlich der Dosis und des zeitlichen Verlaufs der Intensität zu ermitteln und dann entsprechend das System zu steuern.

In ähnlicher Weise kann die Steuerung 7 auch über eine Schnittstelle erhaltene Messdaten hinsichtlich optischer Parameter, also insbesondere hinsichtlich der Wellenfronten und der Topographie, auswerten, um optimale Behandlungsdaten zu ermitteln und das System entsprechend zu steuern.

Auch können analog präoperative und postoperative Messdaten verwendet werden, um für die Behandlung optimale Strahlungsparameter zu berechnen.

Bevorzugt erfolgt die Berechnung von Daten in der Steuerung 7 in Echtzeit (online).

Nachfolgend werden einige Ausgestaltungen des Stativs erläutert:

Das Stativ 16 (Figur 1) dient allgemein zur Positionierung der Bestrahlungseinheit 17 in Bezug auf das zu bestrahlende Gewebe. Zum Beispiel kann es sich um ein Tischstativ handeln. Hierzu kann das Stativ einen Federgelenkarm aufweisen, also einen Arm, der über Federn in einer Ausgangslage als Ruhestellung vorgespannt ist und der aus dieser Ausgangslage durch einen Benutzer verschwenkbar ist und in der verschwenkten Lage dann arretierbar ist. Es ist auch möglich, das mechanische Stativ 16 so zu gestalten, dass es durch Elektromotoren positionierbar ist, ein-dimensional, zwei-dimensional oder drei-dimensional.

Es ist auch möglich, das Stativ 16 direkt mit einem Patientenbett oder Patientenstuhl zu verbinden.

Nachfolgend werden einige Eigenschaften der Benutzerschnittstelle 19 erläutert:
Die Benutzerschnittstelle 19 ermöglicht insbesondere die Eingabe des zeitlichen und räumlichen Verlaufs der Strahlungsintensitäten. Dabei ist insbesondere ein über die Zeit veränderlicher Verlauf der Intensitätsverteilung vorgesehen.

Weiterhin ermöglicht die Benutzerschnittstelle 19 die Eingabe von Patientendaten, wie Hornhautdicke, Epitheldicke, Konzentration und Art des Wirkstoffes 6 im Gewebe, wobei letzterer auch als "Photosensitizer" bezeichnet wird. Auch können über die Benutzerschnittstelle 19 optische Messdaten eingegeben werden.

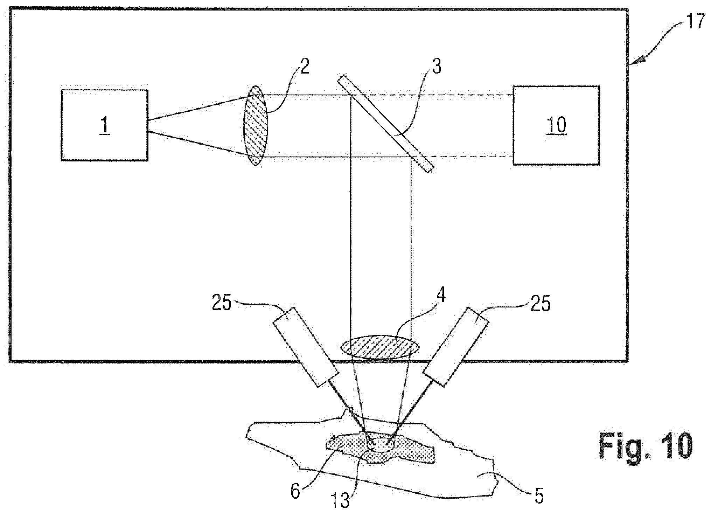

Nachfolgend werden Ausführungsformen gemäß Figur 10 erläutert:
Figur 10 zeigt zwei Lichtquellen 25, die insbesondere als Laserdioden ausgebildet sein können. Diese Strahlen sind ebenfalls auf den Bestrahlungsbereich 13 gerichtet (Figur 10). Sie dienen der räumlichen Justierung und insbesondere Zentrierung des Systems. Die Strahlung der Lichtquellen 25, die vom Gewebe 5 reflektiert wird, kann zum Beispiel über die Linse und den teildurchlässigen Spiegel 3 aufgrund der Wellenlängen separiert werden und mit einem Kamerasystem (an der Stelle des Blockes 9) ausgewertet werden, um eine räumliche Justierung der Strahlung zu ermöglichen. Dafür sind insbesondere der über die Linse 4 auf das Gewebe 5 gerichtete Strahl und der Justierstrahl zumindest einer der Lichtquellen 25 im Sollzustand kozentrisch. Der Winkel, unter dem der Strahl der zumindest einen Lichtquelle 25 (in Figur 10 sind zwei gezeigt) auf das Gewebe 5 auftrifft, ist vorgegeben und genau bekannt.

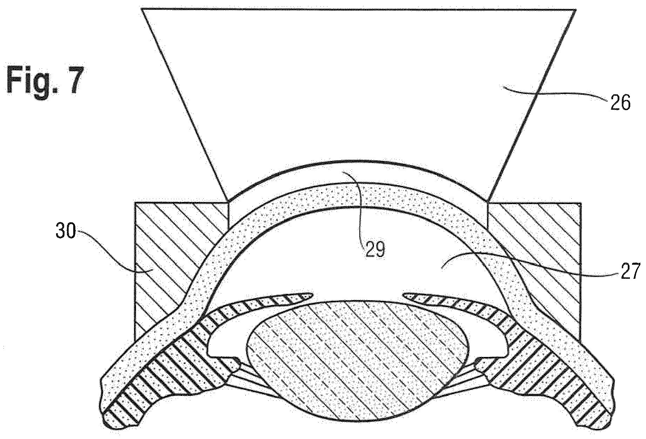

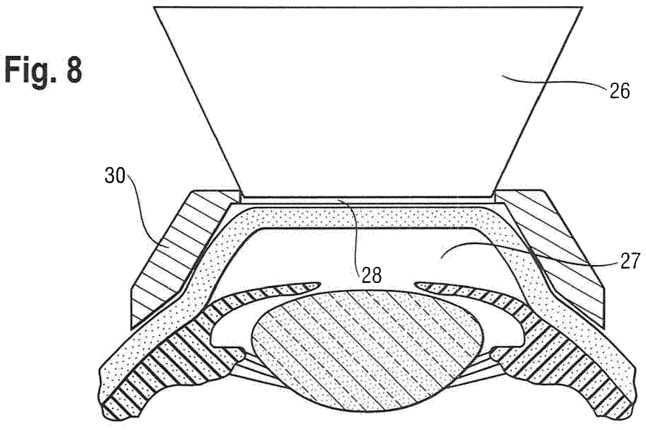

Nachfolgend werden mit Bezugnahme auf die Figuren 7 und 8 einige Einzelheiten einer Einrichtung zum Positionieren des Bestrahlungssystems in Bezug auf ein zu behandelndes Auge erläutert:
Die Bestrahlungseinheit 17 ist über einen Adapter 26 (Figur 7) in Bezug auf das Auge positioniert. Die Figuren 7 und 8 zeigen schematisch die Bestandteile des Auges 27. Der Adapter 26 hat insgesamt eine gesichtsförmige Schale, sodass Kopfbewegungen während der Bestrahlung verhindert sind.

Der Adapter 26 hat weiterhin eine Applanationsform 29, 28 (vgl. Figur 7, Figur 8), die transparent für die verwendete und gegebenenfalls die reflektierte Strahlung ist. Die Applanationsform 28 wird auf die Hornhaut gedrückt und verformt die Hornhaut in gewünschter Weise. Zum Beispiel kann die Form der Applanationsform 28 entsprechend der Diagnose sphärisch, asphärisch bitorisch, oder durch ein Zernike-Polynom beschrieben sein. Das Zernike-Polynom kann bis zur 10. Ordnung gehen.

Der Applikator 26, der die Kornea allseitig umfasst und stützt, kann gemäß einer Ausgestaltung mit Mitteln versehen sein, um das in das Gewebe zu injizierende Medikament abzugeben, und zwar in bestimmten Dosen. Es kann im Applikator eine kleine Pumpe vorgesehen sein, die elektrisch durch die Steuerung 7 ansteuerbar ist, um das Medikament in die Hornhaut zu überführen.

Die Applanationsform des Adapters 26, also die Form, mit dem der Adapter das zu behandelnde Gewebe, also insbesondere die Hornhaut, durch sanftes Andrücken formt, kann so ausgestaltet sein, dass das Gewebe nur in Teilen, also bestimmten ausgewählten Bereichen, geformt wird. Diese geformten Bereiche können innerhalb und/oder außerhalb der bestrahlten Zone liegen.

Der mechanische Adapter 26 kann Sensoren aufweisen, deren Anordnung durch das Bezugszeichen 30 angedeutet ist. Zum Beispiel können die Sensoren biomechanische Eigenschaften des Gewebes ermitteln. Auch könnend die Sensoren 30 vorgesehen sein, um die Konzentration des chemischen Wirkstoffes im Gewebe zu ermitteln.

Auch können die Sensoren 30 ausgebildet sein, um eine Wirkstoffkonzentration in der Vorderkammer des Auges zu ermitteln.

Insgesamt kann der Adapter 26 mit einer mechanischen Ansaugvorrichtung an das Auge 27 versehen sein. Dabei kann ein Sensor vorgesehen sein, um die Aufpresskraft an das Auge zu messen und an die Steuerung 7 ein entsprechendes Signal zu geben.

Auch kann der Adapter 26 mit einem mechanischen System versehen sein, um das Gewebeepithel zu entfernen.

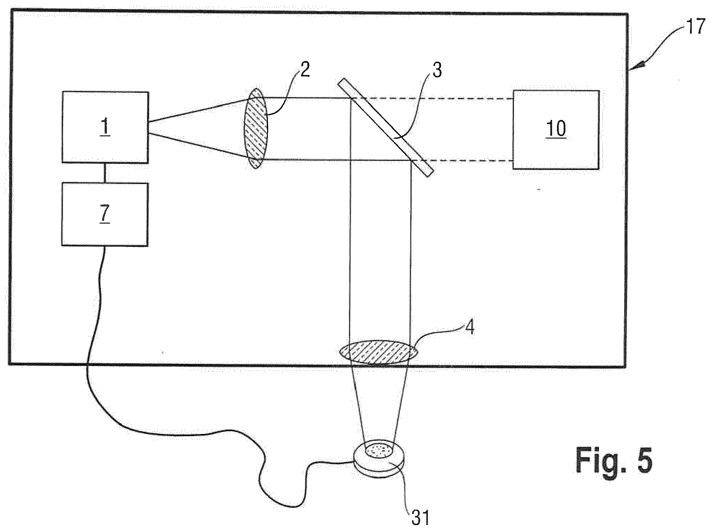

Nachfolgend wird insbesondere mit Blick auf Figur 5 ein externes Kalibriersystem beschrieben:
In der Anordnung gemäß Figur 5 wird eine Bestrahlungseinheit 17 in ihren hier interessierenden Komponenten (ansonsten entspricht sie Figur 1) dargestellt, ohne Zusammenwirken mit einem zu behandelnden Auge. Das Auge ist ersetzt durch ein Kalibriermittel 31. Mit dem Kalibriersystem 31 wird die Funktion des Bestrahlungssystems geprüft, bevor sie am Auge zum Einsatz kommt.

Bei dem Kalibriermittel 31 kann es sich zum Beispiel um einen Energiesensor, ein Spektrometer, eine Strahlprofilkamera, eine Zeitmesseinrichtung, ein Fotometer, oder ein Fluoreszenzmedium für die Wirkungsstrahlung handeln.

Das Kalibriermittel 31 liefert Signale an die Steuerung 7, sodass über die Steuerung ein geschlossener Regelkreis bezüglich der über die Strahlungsquelle 1 abgegebenen Strahlung erzeugbar ist.

Das Kalibriermittel 31 kann auch in den Applikator integriert sein und dann während der Behandlung eingesetzt werden.

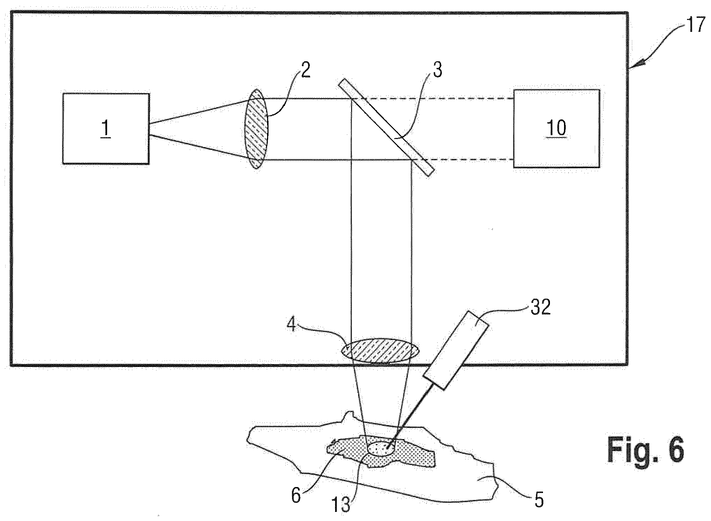

Nachfolgend wird mit Blick auf Figur 6 ein Applikator 32 für Medikamente beschrieben:
Gemäß Figur 6 ist ein Applikator 32 für Medikamente nahe der Bestrahlungsfläche 13 am oder im zu behandelnden Gewebe angeordnet. Bei dem Applikator 32 kann es sich um eine Injektor, ein Tropfsystem, oder ein Spraysystem handeln. Auch kann der Applikator 32 über die Steuerung in seiner Abgabe von Medikamenten gesteuert werden.

Die Steuerung des Applikators 32 über die Steuerung 7 kann bevorzugt in Kombination mit einer Diagnostik während der Behandlung mittels des im Block 9 angeordneten Diagnosemittels erfolgen, also in Form eines geschlossenen Regelkreises.

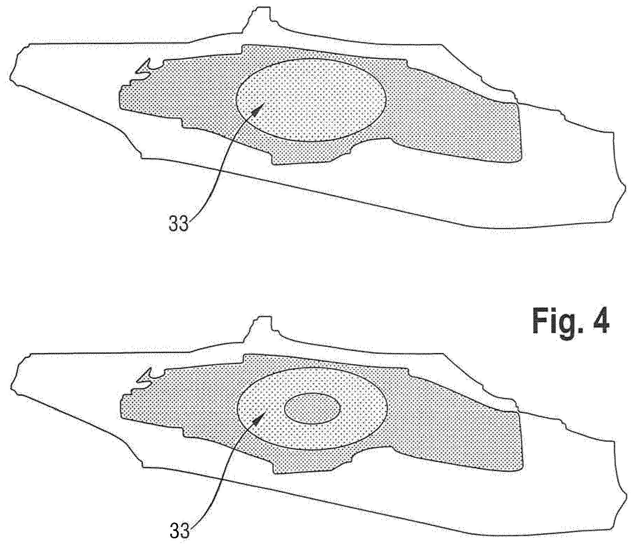

Figur 4 zeigt besondere Ausgestaltungen der Bestrahlung des Gewebes. Der Bestrahlungsbereich 13 kann danach zum Beispiel die in Figur 4 gezeigten besonderen Ausgestaltungen 33 aufweisen, also zum Beispiel eine geschlossene Kreisform gemäß Figur 4, oben, oder ein Kreisringform gemäß Figur 4, unten. Für das Bestrahlungsfeld können auch eine Ellipsenform mit bestimmter Exzentrizität gewählt werden. Die genannten Lichtformen können auch kombiniert werden, zum Beispiel in zeitlicher Abfolge, je nach der Diagnose.

## Patentansprüche

1. Ophthalmologisches Bestrahlungssystem, umfassend:
a. zumindest eine Strahlungsquelle (1), die eine elektromagnetische Strahlung zum Bestrahlen des zu behandelnden Gewebes (5) im Bereich von 300 nm bis 800 nm emittiert, wobei ein Fotosensibilisator in das zu behandelnde Gewebe (5) eingebracht ist, und wobei die elektromagnetische Strahlung fotochemische und/oder fotophysikalische Effekte im Gewebe (5) bewirkt,
b. ein optisches System mit zumindest zwei Linsen (2, 4) und einem Strahlteiler (3), um die Strahlung in einem vorgegebenen Abstand auf das zu bestrahlende Gewebe (5) zu richten,
c. zumindest eine Blende (12), die so ausgelegt und angeordnet ist, dass sie zusammen mit dem optischen System einen Bestrahlungsbereich (13) auf dem Gewebe (13) erzeugt, auf dem die elektromagnetische Strahlung von der zumindest einen Strahlungsquelle fokussiert ist, wobei die zumindest eine Blende (12) steuerbar ist, um den Bestrahlungsbereich (13) anzupassen,
d. ein elektrisches Netzteil zur Energieversorgung der zumindest einen Strahlungsquelle (1),
e. ein Stativ (16) zur Abstützung einer Bestrahlungseinheit (17), welche zumindest die in den Merkmalen a., b., c. genannten Komponenten enthält, in Bezug auf das zu bestrahlende Gewebe (5), wobei es das Stativ (16) gestattet, den Abstand zwischen der Bestrahlungseinheit (17) und dem zu behandelnden Gewebe (5) einzustellen,
f. eine Schnittstelle (19) oder eine Eingabeeinrichtung zur Eingabe von Daten durch einen Benutzer bezüglich Einzelheiten der Behandlung des Gewebes (5) mit dem Fotosensibilisator,
g. eine Steuerung (7) zum Steuern oder Regeln der zumindest einen Strahlungsquelle (1) anhand der eingegebenen Daten bezüglich Einzelheiten der Behandlung des Gewebes (5) mit dem Fotosensibilisator, und
h. eine Anzeigeeinrichtung (18) zum Anzeigen von Behandlungsparametern bezüglich der Behandlung des Gewebes (5) mit dem Fotosensibilisator.

2. Bestrahlungssystem nach Anspruch 1, wobei die zumindest eine Strahlungsquelle (1) zumindest eine LED umfasst.

3. Bestrahlungssystem nach einem der Ansprüche 1 oder 2, wobei die von der Schnittstelle (19) an die Steuerung (7) übermittelten Daten die Bestrahlungsdosis umfassen.

4. Bestrahlungssystem nach einem der Ansprüche 1 bis 3, wobei die Steuerung (7) so programmiert ist, dass sie über die Schnittstelle (19) erhaltene Daten verrechnet, um für die Behandlung optimale Werte hinsichtlich der Dosis oder des zeitlichen Verlaufs der Intensität zu ermitteln und dann entsprechend das System zu steuern.

5. Bestrahlungssystem nach einem der Ansprüche 1 bis 4, wobei der Bestrahlungsbereich (13) eine geschlossene Kreisform, eine Ellipsenform oder eine Kreisringform aufweist.

6. Bestrahlungssystem nach einem der Ansprüche 1 bis 5, ferner umfassend eine Messeinrichtung (10), wobei der Strahlteiler (3) ausgelegt und angeordnet ist, einen Teil der von der Strahlungsquelle emittierten Strahlung auszukoppeln, um den ausgekoppelten Teil der Messeinrichtung (10) zuzuführen.

7. Bestrahlungssystem nach Anspruch 6, wobei die Messeinrichtung (10) ein Fotodetektor ist, mit dem die Strahlungsdosis pro Zeiteinheit und über den zeitlichen Verlauf der Behandlung gemessen wird, wobei der Fotodetektor ausgelegt ist, ein Signal an die Steuerung (7) abzugeben, um den zeitlichen Verlauf der elektromagnetischen Strahlung in der Behandlungsebene gemäß einem vorgegebenen Programm zu steuern oder zu regeln.

8. Bestrahlungssystem nach einem der Ansprüche 1 bis 7, wobei der Strahlteiler (3) zum Kombinieren der von der zumindest einen Strahlungsquelle (1) emittierten Strahlung mit Sekundärstrahlung einer anderen Wellenlänge von einer sekundären Strahlungsquelle ausgelegt und angeordnet ist, wobei die Sekundärstrahlung eine Wellenlänge im sichtbaren Bereich aufweist und als Fixationsstrahl oder Zielstrahl dient.

9. Bestrahlungssystem nach einem der Ansprüche 1 bis 8, wobei die Steuerung ausgelegt ist, die zumindest eine Strahlungsquelle (1) derart zu steuern, dass die Strahlungsquelle (1) die Strahlung pulsierend oder kontinuierlich abgibt.

10. Bestrahlungssystem nach einem der Ansprüche 1 bis 9, wobei Mittel (7) vorgesehen sind, um eine zeitlich und/oder räumlich veränderbare Intensitätsverteilung der Strahlung einzustellen.

11. Bestrahlungssystem nach einem der Ansprüche 1 bis 10, wobei der zumindest eine Strahlteiler (3) einen Teil der auf das Gewebe (5) gerichteten Strahlung zu Mess- oder Überwachungszwecken auskoppelt und/oder zu Beobachtungszwecken und/oder für eine Echtzeit-Diagnostik und/oder für die Zusammenführung der genannten Strahlung mit einer weiteren Strahlung anderer Wellenlänge aus einer weiteren Strahlungsquelle eingerichtet ist.

12. Bestrahlungssystem nach einem der Ansprüche 1 bis 11, ferner rumfassend eine elektronische Schnittstelle (8) zwischen der Steuerung (7) und einem externen Rechner zur Übertragung von Daten, wie zum Beispiel Bestrahlungsdauer, Dosis, Lichtverteilung, Messdaten, Daten aus Datenbänken.

13. Bestrahlungssystem nach einem der Ansprüche 1 bis 12, ferner umfassend zumindest einen Sensor für zum Beispiel die Stromaufnahme der Strahlungsquelle, die Temperatur einer oder mehrerer Komponenten des Systems, die Temperatur der Umgebung, die Luftfeuchtigkeit der Umgebung.

## Claims

1. Ophthalmological radiation system, comprising:
a. at least one source of radiation (1), which emits electromagnetic radiation in the range from 300 nm to 800 nm for irradiating the tissue (5) to be treated, in which a photosensitiser is introduced into the tissue (5) to be treated, and in which the electromagnetic radiation causes photochemical and/or photophysical effects in the tissue (5),
b. an optical system with at least two lenses (2, 4) and a beam splitter (3), in order to direct the radiation onto the tissue (5) to be irradiated at a preset distance,
c. at least one aperture (12), which is designed and arranged so that together with the optical system it produces an irradiation area (13) on the tissue (13), on which the electromagnetic radiation of the at least one source of radiation is focused, in which the at least one aperture (12) may be controlled, in order to adjust the irradiation area (13),
d. an electrical power supply for supplying power to the at least one source of radiation (1),
e. a stand (16) for supporting an irradiation unit (17), which contains at least the components indicated in characteristics a., b., c., with reference to the tissue (5) to be irradiated, in which the stand (16) allows the distance between the irradiation unit (17) and the tissue (5) to be treated to be set,
f. an interface (19) or an input device for data to be input by a user with reference to details of the treatment of the tissue (5) with the photosensitiser,
g. a control (7) for controlling or regulating the at least one source of radiation (1) using the data input with reference to details of the treatment of the tissue (5) with the photosensitiser, and
h. a display device (18) for displaying treatment parameters with reference to the treatment of the tissue (5) with the photosensitiser.

2. Radiation system according to claim 1, in which the at least one source of radiation (1) comprises at least one LED.

3. Radiation system according to one of claims 1 or 2, in which the data transferred from the interface (19) to the control (7) comprise the radiation dose.

4. Radiation system according to one of claims 1 to 3, in which the control (7) is programmed so that it calculates the data obtained through the interface (19), in order to determine optimum values for the treatment with regard to the dose or the progression of the intensity over time and then control the system accordingly.

5. Radiation system according to one of claims 1 to 4, in which the irradiation area (13) has a closed circular shape, an elliptical shape or a circular ring shape.

6. Radiation system according to one of claims 1 to 5, also comprising a measuring device (10), in which the beam splitter (3) is designed and arranged to decouple a part of the radiation emitted from the source of radiation, in order to bring the decoupled part to the measuring device (10).

7. Radiation system according to claim 6, in which the measuring device (10) is a photodetector, with which the radiation dose is measured per time unit and over the progression of the treatment over time, in which the photodetector is designed to emit a signal to the control (7), in order to control or regulate the progression of the electromagnetic radiation in the treatment plane over time according to a preset programme.

8. Radiation system according to one of claims 1 to 7, in which the beam splitter (3) is designed and arranged to combine the radiation emitted from the at least one source of radiation (1) with secondary radiation of another wavelength from a secondary source of radiation, in which the secondary radiation has a wavelength in the visible range and serves as a fixing beam or aiming beam.

9. Radiation system according to one of claims 1 to 8, in which the control is designed to control the at least one source of radiation (1) in such a way that the source of radiation (1) emits the radiation in a pulsating or continuous way.

10. Radiation system according to one of claims 1 to 9, in which means (7) are provided, in order to set an intensity distribution of the radiation that may be changed in terms of time and/or space.

11. Radiation system according to one of claims 1 to 10, in which the at least one beam splitter (3) decouples a part of the radiation directed onto the tissue (5) for measuring or monitoring purposes and/or for observation purposes and/or is set up for real time diagnosis and/or for bringing together the radiation indicated with further radiation of another wavelength from a further source of radiation.

12. Radiation system according to one of claims 1 to 11, also comprising an electronic interface (8) between the control (7) and an external computer for transferring data, such as duration of irradiation, dose, light distribution, measuring data and data from data bases for example.

13. Radiation system according to one of claims 1 to 12, also comprising at least one sensor for the power consumption of the source of radiation, temperature of one or several components of the system, temperature of the environment and atmospheric humidity of the environment for example.

## Revendications

1. Système d'irradiation ophtalmologique comprenant :
a. au moins une source de rayonnement (1) qui émet un rayonnement électromagnétique pour l'irradiation du tissu à traiter (5) de l'ordre de 300 nm à 800 nm, un photosensibilisateur étant inséré dans le tissu à traiter (5) et le rayonnement électromagnétique provoquant des effets photochimiques et/ou photophysiques dans le tissu (5),
b. un système optique avec au moins deux lentilles (2, 4) et un diviseur de faisceau (3) afin d'orienter le rayonnement à une distance prédéterminée vers le tissu à traiter (5),
c. au moins un diaphragme (12) qui est conçu et disposé de façon à générer, conjointement avec le système optique, une zone d'irradiation (13) sur le tissu (13), sur laquelle le rayonnement électromagnétique provenant de l'au moins une source de rayonnement est focalisé, l'au moins un diaphragme (12) pouvant être contrôlé afin d'adapter la zone d'irradiation (13),
d. un bloc d'alimentation électrique pour l'alimentation en énergie de l'au moins une source de rayonnement (1),
e. un trépied (16) pour le soutien d'une unité d'irradiation (17), qui contient au moins les composants mentionnés aux caractéristiques a., b., c., par rapport au tissu à traiter (5), moyennant quoi cela permet au trépied (16) de régler la distance entre l'unité d'irradiation (17) et le tissu à traiter (5),
f. une interface (19) ou un dispositif d'entrée pour l'entrée de données par un utilisateur concernant les détails du traitement du tissu (5) avec le photosensibilisateur,
g. une commande (7) pour le contrôle ou la régulation de l'au moins une source de rayonnement (1) à l'aide des données entrées concernant les détails du traitement du tissu (5) avec le photosensibilisateur et
h. un dispositif d'affichage (18) pour l'affichage de paramètres de traitement concernant le traitement du tissu (5) avec le photosensibilisateur.

2. Système d'irradiation selon la revendication 1, l'au moins une source de rayonnement (1) comprenant au moins une LED.

3. Système d'irradiation selon l'une des revendications 1 ou 2, les données transmises par l'interface (19) à la commande (7) comprenant la dose d'irradiation.

4. Système d'irradiation selon l'une des revendications 1 à 3, la commande (7) étant programmée de façon à ce qu'elle compense les données obtenues par l'intermédiaire de l'interface (19) afin de déterminer des valeurs optimales pour le traitement en ce qui concerne la dose ou le tracé temporel de l'intensité puis pour contrôler le système en conséquence.

5. Système d'irradiation selon l'une des revendications 1 à 4, la zone d'irradiation (13) présentant une forme circulaire fermée, une forme elliptique ou une forme annulaire.

6. Système d'irradiation selon l'une des revendications 1 à 5, comprenant en outre un dispositif de mesure (10), le diviseur de faisceau (3) étant conçu et disposé pour faire sortir une partie une partie du rayonnement émis par la source de rayonnement afin d'introduire la partie sortie dans le dispositif de mesure (10).

7. Système d'irradiation selon la revendication 6, le dispositif de mesure (10) étant un photodétecteur avec lequel la dose de rayonnement par unité de temps et sur le tracé temporel du traitement, est mesurée, le photodétecteur étant conçu pour envoyer un signal à la commande (7) afin de contrôler ou de réguler le tracé temporel du rayonnement dans le plan de traitement selon un programme prédéterminé.

8. Système d'irradiation selon l'une des revendications 1 à 7, le diviseur de faisceau (3) étant conçu et disposé pour la combinaison du rayonnement émis par l'au moins une source de rayonnement (1) avec un rayonnement secondaire d'une autre longueur d'onde provenant d'une source de rayonnement secondaire, le rayonnement secondaire présentant une longueur d'onde dans le domaine visible et servant de rayon de fixation ou de rayon de visée.

9. Système d'irradiation selon l'une des revendications 1 à 8, la commande étant conçue pour contrôler l'au moins une source de rayonnement (1) de façon à ce que la source de rayonnement (1) émette le rayonnement de manière pulsée ou de manière continue.

10. Système d'irradiation selon l'une des revendications 1 à 9, des moyens (7) étant prévus pour régler une répartition d'intensité variable du rayonnement dans le temps et/ou dans le temps.

11. Système d'irradiation selon l'une des revendications 1 à 10, l'au moins un diviseur de faisceau (3) faisant sortir une partie du rayonnement dirigé vers le tissu (5) à des fins de mesure ou de surveillance et/ou à des fins d'observation et/ou pour un diagnostic en temps réel et/ou pour la fusion du rayonnement mentionné avec un autre rayonnement d'une autre longueur d'onde provenant d'une autre source de rayonnement.

12. Système d'irradiation selon l'une des revendications 1 à 11, comprenant en outre une interface électronique (8) entre la commande (7) et un ordinateur externe pour la transmission de données, comme la durée d'irradiation, la dose, la répartition de la lumière, des données de mesure, des données provenant de bases de données.

13. Système d'irradiation selon l'une des revendications 1 à 12, comprenant en outre au moins un capteur pour, par exemple la consommation de courant de la source de rayonnement, la température d'un ou plusieurs composants du système, la température ambiante, l'humidité de l'air ambiant.
